(19) Europäisches Patentamt
European Patent Office
Office européen des brevets

(11) Veröffentlichungsnummer : **0 379 109 B1**

(12) **EUROPÄISCHE PATENTSCHRIFT**

(45) Veröffentlichungstag der Patentschrift : **23.06.93 Patentblatt 93/25**

(51) Int. Cl.$^5$ : **C07C 229/24,** C11D 3/33

(21) Anmeldenummer : **90100696.5**

(22) Anmeldetag : **13.01.90**

# (54) Glycerinaminocarboxylate, ihre Herstellung und Verwendung.

(30) Priorität : **20.01.89 DE 3901613**

(43) Veröffentlichungstag der Anmeldung : **25.07.90 Patentblatt 90/30**

(45) Bekanntmachung des Hinweises auf die Patenterteilung : **23.06.93 Patentblatt 93/25**

(84) Benannte Vertragsstaaten : **AT BE CH DE DK ES FR GB IT LI NL SE**

(56) Entgegenhaltungen :
**EP-A- 0 287 846**
**EP-A- 0 287 885**
**GB-A- 1 444 874**
**US-A- 4 021 359**

(73) Patentinhaber : **BASF Aktiengesellschaft**
**Carl-Bosch-Strasse 38**
**W-6700 Ludwigshafen (DE)**

(72) Erfinder : **Oftring, Alfred, Dr.**
**Im Roehrich 49**
**W-6702 Bad Duerkheim (DE)**
Erfinder : **Birnbach, Stefan, Dr.**
**Budapester Strasse 45**
**W-6700 Ludwigshafen (DE)**
Erfinder : **Fikentscher, Rolf, Dr.**
**Von-Stephan-Strasse 27**
**W-6700 Ludwigshafen (DE)**
Erfinder : **Baur, Richard, Dr.**
**Nelkenstrasse 1**
**W-6704 Mutterstadt (DE)**
Erfinder : **Kud, Alexander, Dr.**
**Am Hellbrunn 57**
**W-6509 Eppelsheim (DE)**
Erfinder : **Goeckel, Ulrich, Dr.**
**Leipziger Strasse 6**
**W-6737 Beohl-Iggelheim (DE)**
Erfinder : **Perner, Johannes, Dr.**
**Ginsterweg 4**
**W-6730 Neustadt (DE)**

**EP 0 379 109 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Aus der US-PS 4 021 359 sind Waschmittelformulierungen bekannt, die 5 bis 50 Gew.% einer waschaktiven Substanz und einen Builder enthalten, der durch praktisch vollständige Veresterung von beispielsweise Pentaerythrit, Sorbit oder Mannit mit Maleinsäureanhydrid und Neutralisation der Veresterungsprodukte hergestellt wird.

Aus der GB-PS 1 444 874 ist eine Reinigungsmittelmischung bekannt, bei der Phosphate ganz oder teilweise durch Reaktionsprodukte ersetzt sind, die durch Veresterung von mindestens dreiwertigen Alkoholen oder Sacchariden mit Dicarbonsäureanhydriden und anschließende Neutralisation der Veresterungsprodukte erhalten werden und die mindestens drei Estergruppierungen aufweisen. Aus der EP-A-0 287 885 ist u.a. bekannt, Serin-N,N-diessigsäure und ihre Derivate als Komplexbildner für Schwermetall- und/oder Erdalkalimetallionen zu verwenden.

Der vorliegenden Erfindung liegt die Aufgabe zugrunde, wirksamere Komplexbildner für Schwermetall- und Erdalkalimetallionen zur Verfügung zu stellen, die außer einen guten komplexbildenden Wirkung aus ökologischer Sicht unbedenklich sind.

Die Aufgabe wird erfindungsgemäß gelöst mit Glycerinaminocarboxylaten der Formel

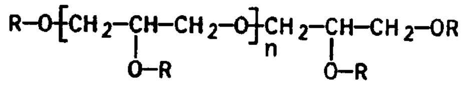 (I),

in der
n = 0 bis 10 bedeutet und
R für

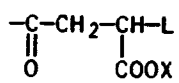 oder 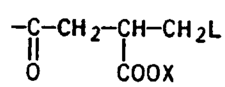

steht, wobei

L einen Iminodiacetat-, Aspartat-, Glutamat-, Sarcosinat-, Glycinat-, Serinat-, Hydroxyaspartat-, Ethanolaminoacetat-, Diethanolamino-, Alanat- oder Taurinatrest darstellt und
X für Wasserstoff-, Alkalimetall-, Ammonium- oder substituiertes Ammoniumion steht.

Die Verbindungen der Formel I sind dadurch erhältlich, daß man

(a) Verbindungen der Formel

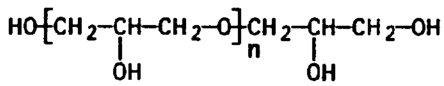 (II),

in der n = 0 bis 10 bedeutet, mit

(b) Maleinsäureanhydrid, Itaconsäureanhydrid, einem Maleinsäurehalbester oder einem Itaconsäurehalbester zu Verbindungen der Formel

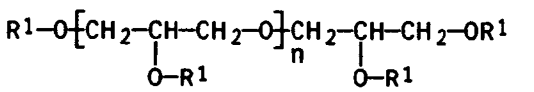 (III),

in der
$R^1$ für

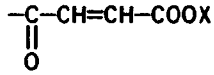 oder 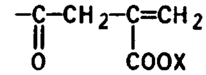

steht und X die in Formel I angegebene Bedeutung hat, verestert und anschließend die Verbindungen der Formel III mit Verbindungen der Formel

L - H (IV),

in der L die im Anspruch 1 angegebene Bedeutung hat, im schwach alkalisch wässrigen Milieu zu Verbindungen der Formel I umsetzt.

Zur Herstellung der erfindungsgemäßen Verbindungen der Formel I erfolgt in einer ersten Reaktionsstufe die Umsetzung der oben angegebenen Verbindungen (a) mit (b). Die Verbindungen der Gruppe (a) können mit Hilfe der Formel

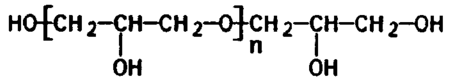

(II)

charakterisiert werden, in der n = 0 bis 10, vorzugsweise 1 bis 4 ist. Geeignete Verbindungen (a) sind beispielsweise Glycerin, Diglycerin, Triglycerin, Tetraglycerin, Pentaglycerin, Hexaglycerin, Heptaglycerin, Octaglycerin und Decaglycerin. Vorzugsweise kommen diejenigen Polyglycerine der Formel II in Betracht, in der n = 1 bis 4 bedeutet. Polyglycerine der angegebenen Art sind bekannt. Sie werden durch Kondensation von Glycerin bei Temperaturen von etwa 200 bis 250°C hergestellt. Diese Kondensationsreaktion wird vorzugsweise durch Zusatz von Säuren oder von Basen katalysiert. Dabei entstehen Polyglycerine, die noch etwa bis zu 20 Gew.% an Glycerin enthalten können sowie Polyglycerine, die höhere Kondensationsgrade aufweisen. Die Viskositäten der so erhältlichen Polyglycerine betragen etwa 500 bis 1500 mPas. Die Kondensationsprodukte haben OH-Zahlen von 1500 bis 1000 mg KOH/g.

Geeignete Verbindungen der Gruppe (b) sind Maleinsäureanhydrid, Itaconsäureanhydrid sowie die Halbester von Maleinsäure und Itaconsäure, die sich jeweils von einwertigen $C_1$- bis $C_4$-Alkoholen ableiten, z.B. Maleinsäuremonomethylester, Maleinsäuremonoethylester, Maleinsäuremono-n-propylester, Maleinsäuremonoisopropylester, Maleinsäuremono-n-butylester, Maleinsäuremono-isobutylester sowie die entsprechenden Halbester der Itaconsäure.

Die Umsetzung der Verbindungen der Gruppen (a) und (b) zu den Verbindungen der Formel

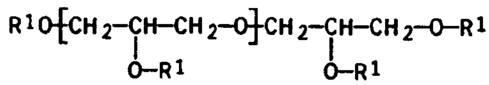

(III),

in der
$R^1$ =

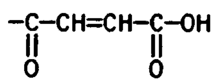

oder

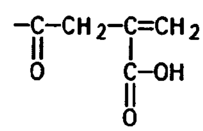

bedeutet, erfolgt bei Temperaturen von 80 bis 140°C. Die Veresterung der Verbindungen (a) wird vorzugsweise in Gegenwart eines Veresterungskatalysators, z.B. Natriumacetat, durchgeführt. Pro OH-Moläquivalent (a) setzt man 0,05 bis 1,5, vorzugsweise 1 bis 1,1 mol einer Verbindung (b) ein. Die Herstellung von Polyglycerin und die Veresterung des Polyglycerins mit den Verbindungen (b) kann besonders vorteilhaft in einem einzigen Reaktions-gefäß erfolgen, indem man darin zunächst Glycerin bei Temperaturen von 200 bis 250°C kondensiert, danach das Polyglycerin auf eine Temperatur in dem Bereich von 80 bis 140, vorzugsweise 100 bis 120°C abkühlt und dann durch Zugabe der oben angegebenen Mengen an Verbindungen der Gruppe (b) in Abwesenheit von Lösemitteln sowie vorzugsweise unter Ausschluß von Wasser verestert. Besonders bevorzugt bei der Veresterung ist eine Arbeitsweise, bei der man beispielsweise das Maleinsäureanhydrid unter die Oberfläche eines auf 80 bis 140°C erwärmten Polyglycerins dosiert. Dadurch wird nämlich eine Sublimation von Maleinsäureanhydrid praktisch vollständig unterdrückt. Bei dieser Verfahrensweise erhält man Umsetzungsgrade von mehr als 90 %. Eine Vervollständigung der Umsetzung wird dadurch erreicht, daß man das Reaktionsgemisch bei den angegebenen Umsetzungstemperaturen im Anschluß an die Zugabe des Maleinsäureanhydrids eine bestimmte Zeit lang rührt, z.B. 1 bis 2 Stunden. Beim Abkühlen der Verbindungen der Formel III auf Temperaturen unterhalb von 50°C erhält man eine glasartig erstarrte Masse. Die Verbindungen der Formel III sind jedoch in dem Temperaturbereich von 80 bis 100°C noch gut rührbar. Um die Verbindungen der Formel III gut handhaben zu können, setzt man zu der Schmelze der Verbindungen der Formel III in dem Temperaturbereich von 80 bis 130°C soviel Wasser zu, daß man klare 50 bis 80 gew.%ige wäßrige Lösungen der Verbindungen der Formel III erhält. Überraschenderweise tritt bei einer derartigen Herstellung von wäßrigen konzentrierten Lösungen der Verbindungen der Formel III praktisch keine merkliche Verseifung der Esterfunktionen ein, obwohl die Temperaturen bei der Herstellung der wäßrigen Lösung 80 bis 130°C betragen. Besonders bevorzugt wegen der sehr guten biologischen Abbaubarkeit sind diejenigen Glycerinaminocarboxylate, die durch Veresterung von Verbindungen der obengenannten Formel II mit Maleinsäureanhydrid bei Temperaturen von 80 bis 140°C unter Ausschluß von Wasser, Zugabe von Wasser zur Schmelze der Ester unter Bildung 50 bis 80 gew.-%iger Lösungen und Umsetzung mit Glutaminsäure erhältlich sind.

Setzt man bei der Veresterung der Verbindungen (a) Maleinsäurehalbester oder Itaconsäurehalbester ein, so benutzt man die üblichen Umesterungskatalysatoren, wie beispielsweise Titantetraalkyl-Verbindungen und destilliert während der Umesterung der Maleinsäure- bzw. Itaconsäurehalbester die jeweils entstehenden $C_1$- bis $C_4$-Alkohole kontinuierlich aus der Reaktionsmischung ab. Besonders bevorzugt ist die Herstellung von Verbindungen der Formel III, in der praktisch sämtliche OH-Gruppen der zugrundeliegenden Verbindungen der Gruppe (a) verestert sind und bei denen die Veresterung mit Maleinsäureanhydrid oder einem Maleinsäurehalbester vorgenommen worden ist. Zur Herstellung dieser Verbindungen setzt man die Verbindungen der Formel II vorzugsweise mit Maleinsäureanhydrid oder Maleinsäuremono-$C_1$- bis $C_4$-alkylester um, wobei man pro OH-Äquivalent der Verbindung II 1 bis 1,5 Moläquivalente Maleinsäureanhydrid oder Maleinsäuremono-$C_1$-bis $C_4$-alkylester einsetzt.

Die erfindungsgemäßen Verbindungen der Formel I werden in einer zweiten Stufe der Umsetzung durch Reaktion der Verbindungen der Formel III mit den Verbindungen der Gruppe (c) erhalten. Zu der Gruppe (c) gehören Amine und Aminosäuren der Struktur L-H. Geeignete Verbindungen der Gruppe (c) sind beispielsweise Ethanolamin, Diethanolamin, Iminodiessigsäure, Asparaginsäure, Glutaminsäure, Ethanolaminoessigsäure, Hydroxyasparaginsäure, Sarcosin, Glycin, Serin, Taurin und Laurin. Von den genannten Verbindungen verwendet man vorzugsweise Iminodiessigsäure und Glutaminsäure.

Die Addition der Verbindungen (c) an die in den Verbindungen der Formel III enthaltenen Doppelbindungen erfolgt in wäßriger Lösung. Die Verbindungen (c) werden dabei in einer Menge eingesetzt, daß pro Moläquivalent an Doppelbindungen in der Formel III 0,5 bis 1,3 Moläquivalente der Amine oder Aminosäuren der Struktur L-H, vorzugsweise 0,9 bis 1,1 Moläquivalente angewendet werden. Da die Verbindungen der Formel III bereits in wäßriger Lösung vorliegen, kann die Zusammenführung der einzelnen Komponenten beliebig variiert werden. So können beispielsweise wäßrige Lösungen von Verbindungen der Formel III und wäßrige Lösungen der Verbindungen der Gruppe (c) jeweils getrennt in ein Reaktionsgefäß dosiert und darin umgesetzt werden. Man kann jedoch auch so vorgehen, daß man wäßrige Lösungen von Verbindungen der Formel III in einem Reaktionsbehälter vorlegt und die Verbindungen der Gruppe (c) absatzweise oder kontinuierlich zugibt. Bei dieser Verfahrensweise können sowohl Temperatur als auch pH-Wert leicht kontrolliert und konstant gehalten werden. Vorzugsweise wird in der zweiten Stufe zur Herstellung der erfindungsgemäßen Verbindungen mindestens eine Verbindung der Komponente (c) vorgelegt und die wäßrige Lösung der Verbindungen der Formel (III) unter kontrollierten Bedingungen zugegeben. Die Umsetzung erfolgt vorzugsweise im schwach alkalischen Milieu bei pH-Werten von 8 bis 11, vorzugsweise 9 bis 10. Die Reaktionstemperatur beträgt 10 bis 70, vorzugsweise 15 bis 40°C. Es ist vorteilhaft, die Reaktion in einem gepufferten Milieu vorzunehmen. Als Puffersubstanzen kommen beispielsweise Natriumcarbonat und Natriumacetat in Betracht.

Da die zweite Reaktionsstufe in alkalischem Milieu abläuft, empfiehlt es sich, die Verbindungen der Formel III vor der Umsetzung mit den Verbindungen (c) zu neutralisieren. Die Neutralisation wird vorzugsweise bei Temperaturen von unterhalb 50°C, z.B. in dem Bereich von 0 bis 45, vorzugsweise 10 bis 25°C vorgenommen. Als Neutralisationsmittel setzt man Basen ein, z.B. Alkalilaugen, vorzugsweise Natronlauge oder Kalilauge, Ammoniak, Amine, wie Triethylamin, Tributylamin, Ethanolamin, Diethanolamin, Triethanolamin und Morpholin. Die Menge an Puffersubstanzen beträgt, bezogen auf die insgesamt für die Neutralisation der Carboxylgruppen der Verbindungen der Formel III erforderlichen Menge an Basen, 5 bis 40, vorzugsweise 10 bis 25 mol.% an Natriumcarbonat bzw. Natriumacetat. Die Konzentration der Feststoffe in der zweiten Stufe der Herstellung der erfindungsgemäßen Verbindungen beträgt 30 bis 70, vorzugsweise 45 bis 60 Gew.%.

Bei der Umsetzung der Verbindungen der Formeln III und IV erhält man die Verbindungen der Formel I

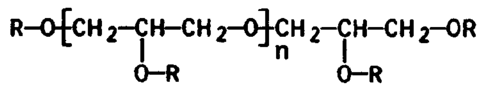 ,

in der n = 0 bis 10, vorzugsweise 1 bis 4 ist,
R =

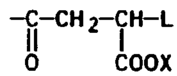 oder 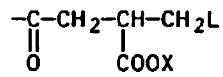

bedeutet,
X = ein Wasserstoff-, Alkalimetall-, Ammonium- oder substituiertes Ammoniumionäquivalent und
L =

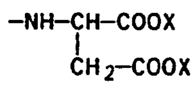 (Aspartat) - (1)

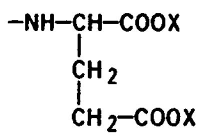 (Glutamat) - (2)

-NH-$CH_2$-COOX (Glycinat) - (3)

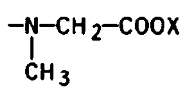 (Sarkosinat) - (4)

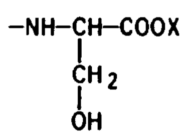 (Serinat) - (5)

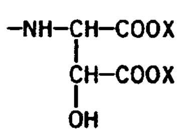 (Hydroxyaspartat) - (6)

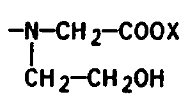 (Ethanolaminoacetat) - (7)

-NH-$CH_2$-$CH_2$OH (Ethanolamino) - (8)

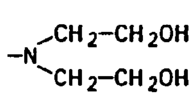 (Diethanolamino) - (9)

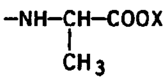 (Alanat) - (10)

$-NH-CH_2-CH_2-SO_3X$ (Taurinat) - (11)

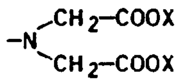 (Iminodiacetat) - (12)

Die Verbindungen der Formel I können modifiziert werden, beispielsweise in der Weise, daß sie zusätzlich noch Sulfogruppen enthalten. Solche Verbindungen sind dadurch herstellbar, daß man bis zu 30 Gew.% der Verbindungen (c) durch Sulfit oder Bisulfit, vorzugsweise in der Form der Natrium- oder Kaliumsalze ersetzt. Dadurch erreicht man eine Addition von Natriumsulfit bzw. Kaliumsulfit an die Doppelbindung der in den Verbindungen III enthaltenen Estergruppierungen.

Eine weitere Modifizierung der erfindungsgemäßen Stoffe der Formel I ist dadurch möglich, daß man einen Teil der OH-Gruppen des Glycerins oder Polyglycerins, z.B. bis zu 30 mol.% der OH-Gruppen, mit einer aliphatischen oder aromatischen Carbonsäure anstelle von Maleinsäure oder Itakonsäure verestert. Beispielsweise eignen sich hierfür langkettige Carbonsäuren, z.B. 2-Ethylhexansäure oder $C_4$- bis $C_{18}$-Carbonsäuren, wie Stearinsäure oder aromatische Carbonsäuren, wie Phthalsäure. Auch partiell mit Ethylenoxid umgesetzte Polyglycerine oder Glycerin können als Verbindungen der Gruppe (a) zur Modifizierung der Verbindungen der Formel I eingesetzt werden. Bei einer Ethoxylierung der Verbindungen der Formel II beträgt die Menge an Ethylenoxid bis zu 50 mol.%.

Die Glycerinaminocarboxylate der Formel I können in verschiedener Weise aufgearbeitet werden, beispielsweise kann man die Natriumsalzlösungen der Verbindungen der Formel I durch Zusatz von Schwefelsäure bzw. durch Einleiten von Kohlendioxid in die Säureform überführen. Die Natriumsalze können direkt aus den wäßrigen Lösungen durch Abdestillieren des Wasser, vorzugsweise unter vermindertem Druck, oder durch Sprühtrocknung in reiner Form erhalten werden. Die Salze der Verbindungen der Formel I, z.B. die Natriumsalze, können auch dadurch isoliert werden, daß man zu der wäßrigen Lösung der Natriumsalze der Verbindungen der Formel I die zwei- bis dreifache Menge eines mit Wasser mischbaren organischen Lösemittels zusetzt, z.B. Methanol oder Aceton. Dadurch fallen die Natriumsalze aus und können leicht isoliert werden.

Die Verbindungen der Formel I werden als Komplexbildner für Schwermetall- und/oder Erdalkalimetallionen verwendet. Die Verbindungen eignen sich insbesondere zur Komplexierung von Kalzium, Magnesium-, Eisen-, Kupfer-, Nickel- und Manganionen. Aufgrund dieser Eigenschaft können sie auf verschiedenen technischen Gebieten angewendet werden. Von besonderem Vorteil ist hierbei, daß es sich bei den erfindungsgemäßen Komplexbildnern um biologisch sehr gut abbaubare Verbindungen handelt. Die Verbindungen der Formel I können beispielsweise als Bestandteil von Redoxkatalysatoren verwendet werden, um das Ausfallen der in den Redoxkatalysatoren üblicherweise verwendeten Eisensalz im alkalischen Milieu zu verhindern.

Außer der komplexbildenden Eigenschaft besitzen die Verbindungen der Formel I dispergierende Wirkung. Sie können daher als Dispergiermittel für feinteilige Niederschläge, insbesondere bei der Wasserbehandlung als Scale-Inhibitor eingesetzt werden. Die Anwendungsmengen betragen ca. 0,1 bis 100 ppm, bezogen auf Wasser.

In der photographischen Industrie können die Verbindungen der Formel I in Entwickler- und Fixierbädern eingesetzt werden, zu deren Herstellung hartes Wasser verwendet wurde. Die Verbindungen der Formel I verhindern darin die Ausfällung schwer löslicher Kalzium- und Magnesium-Salze. Eisen-III-Komplexbildnerlösungen können vorteilhaft in Bleichfixierbädern eingesetzt werden, in denen sie die aus ökologischen Gründen bedenklichen Hexacyanoferratlösungen ersetzen können. Die erfindungsgemäßen Verbindungen der Formel I können außerdem in der Textilindustrie zur Entfernung von Schwermetallspuren während des Herstellungs- bzw. Färbeprozesses von natürlichen und synthetischen Fasern dienen. Dadurch werden viele Störungen verhindert, Schmutz, Flecken und Streifen auf dem Textilgut, Verlust des Glanzes, schlechte Benetzbarkeit und unegale Färbungen. Beim Einsatz der erfindungsgemäßen Verbindungen als Komplexbildner in der Papierindustrie werden Störungen durch Schwermetallionen, insbesondere Eisenionen bei der Papierherstellung verhindert.

Sie können auch als Dispergiermittel für Pigmente Verwendung finden, z.B. bei der Herstellung von wäßrigen, hochkonzentrierten Anschlämmungen von Clay oder Kreide für die Bereitung von Papierstreichmassen.

Die Verbindungen der Formel I eignen sich außerdem als Komplexbildner in wäßrigen Bädern für die chemische Abscheidung von Kupfer. Die Bäder zur chemischen Verkupferung sind wäßrige Lösungen, die üblicherweise ein Kupfersalz, Formaldehyd, ein Alkalihydroxid und einen oder mehrere Komplexbildner und ggf. weitere Hilfsmittel enthalten. Der Komplexbildner hat dabei die Aufgabe, das Ausfallen von Kupferhydroxid während der Verkupferung zu verhindern und die Konzentration an freien Kupferionen soweit herabzusetzen, daß eine unbeabsichtigte sogenannte wilde Kupferausfällung vermieden wird. Zusätzlich können die Bäder beispielsweise noch Stabilisatoren in der Größenordnung von einem bis einigen mg/Liter enthalten. Solche Stabilisatoren sind beispielsweise Natriumcyanid, Allylthioharnstoff oder 2,2'-Dipyridylamin. Bei der Verkupferung betragen die Temperaturen in der Regel 15 bis 50, bevorzugt 20 bis 30°. Die Verkupferung ist innerhalb eines Zeitraums von 5 bis 60, vorzugsweise 10 bis 40 Minuten beendet. Die Konzentration an Komplexbildnern der Formel (I) in den Bädern beträgt etwa 10 bis 40 g/Liter Badflüssigkeit. Der pH-Wert der Bäder beträgt mindestens 10. Als Kupferverbindungen kommt vorzugsweise kristallines Kupfersulfat oder Kupfer-II-chlorid in Betracht. Formaldehyd wird in Mengen von 1 bis 12 g/Liter zugesetzt. Die Bäder können ggf. außer den oben bereits erwähnten Stabilisatoren noch übliche Tenside enthalten.

Die in den Beispielen angegebenen Hydrieriodzahlen wurden nach DIN 53 241, Teil 2, bestimmt. Die Prozentangaben in den Beispielen beziehen sich auf das Gewicht der Stoffe. Die K-Werte wurden nach H. Fikentscher, Cellulosechemie, Band 13, 58 bis 64 und 71 bis 74 (1932) bestimmt; dabei bedeutet $K = k \cdot 10^3$. Die Messungen erfolgten in wäßriger Lösung bei 25°C, einem pH-Wert von 7,0 und einer Polymerkonzentration von 1 Gew.% an Na-Salz der Polymerisate.

## Beispiele

### Beispiel 1

a) Herstellung von Polyglycerin-Maleinsäure-Ester (PGN-MS-Ester)

185 g eines Polyglycerins mit einem mittleren Kondensationsgrad $\bar{n}$ = 2,8 (OH-Zahl = 1176 mg KOH/g) wurden auf 120 °C erwärmt. In einer Stickstoffatmosphäre wurden anschließend 392 g (4 Mol) flüssiges Maleinsäureanhydrid, das eine Temperatur von 70°C hatte, innerhalb von 2,5 Stunden unter die Flüssigkeitsoberfläche des Polyglycerins dosiert. Anschließend rührte man 1 Stunde bei 120°C weiter. Es resultierte ein gelbes Öl (Analytik: Verseifungszahl 788 mg KOH/g, Säurezahl 457), das bei 100°C mit 250 g Wasser unter kräftigem Rühren versetzt wurde. Es resultierten 771 g einer 70 %igen wässrigen hellgelben Lösung von PGN-MS-Ester, der eine Verseifungszahl von 774 und eine Säurezahl von 451 (jeweils berechnet 100 %) aufwies. Der Gehalt an freier Maleinsäure betrug nach HPLC-Analyse 3,9 %; Hydrierjodzahl: 186.

b) Umsetzung von PGN-MS-Ester aus 1a mit Iminodiessigsäure (IDA) zu PGN-MS-IDA

226 g (1,7 Mol) Iminodiessigsäure wurden in 250 g Wasser suspendiert. Mit 289 g 40 %iger wäßriger Natronlauge wurde ein pH-Wert von 10 eingestellt. Nach Zugabe von 90 g Soda wurden bei einer Temperatur von 10 bis 15 °C unter kräftigem Rühren 422 g der 70 %igen PGN-MS-Esterlösung innerhalb von 45 Minuten zugetropft. Hierbei wurde der pH-Wert durch Zugabe von 219 g 40 %iger Natronlauge bei pH 10 gehalten. Anschließend wurde 2 Stunden bei 25 °C gerührt. Entspechend der HPLC-Analytik enthielt die resultierende Lösung als Nebenkomponenten noch 2,5 % Iminodiessigsäure-Natriumsalz, 0,5 % Fumarsäure-Natrium, 1,4 % Maleinsäure-Natrium sowie als Verseifungsprodukt des PGN-MS-IDA-Endproduktes 0,4 % Asparaginsäurediessigsäure-Natrium.

Aufarbeitung: Das PGN-MS-IDA-Produkt kann beispielsweise durch Zugabe von Methanol ausgefällt werden. Eine so hergestellte Ware enthielt als Na-Salz-Nebenbestandteile 3,9 % IDA, 0,4 Fumarsäure, 1,1 % Maleinsäure sowie 1,5 % Asparaginsäurediessigsäure. Im IR-Spektrum (KBr-Pressling) sind Banden bei 1720 $cm^{-1}$ (CO-Ester-Schwingung) und bei 1600 $cm^{-1}$ (Carboxylat-, Amincarboxylat-Schwingung) vorhanden. Hydrierjodzahl: 10.

### Beispiel 2

Die Umsetzung wurde mit einem PGN-MS-Ester durchgeführt, der analog zu Beispiel 1a erhalten wurde. Die resultierende 70 %ige wässrige Lösung wurde anschließend unter Kühlung bei 10 bis 15 °C mit 40 %ger wäßriger NaOH auf pH 7 gestellt. Danach wurde in diesem Temperaturintervall eine wässrige neutralisierte Lösung einer bezüglich der vorhandenen Estergruppen äquimolaren IDA-Menge innerhalb von 30 Minuten zugetropft. Anschließend wurde durch Zugabe der Soda- sowie der restlichen Natronlaugemenge ein pH-Wert von 10 eingestellt und die Reaktion wie unter 1b beschrieben fortgeführt. Die resultierende Natriumsalzlösung enthielt als Nebenkomponenten 3,7 % IDA, 0,4 % Fumar- und 2,3 % Maleinsäure sowie 1,1 % Asparaginsäurediessigsäure in Form der Natriumsalze.

Beispiel 3

Analog Beispiel 1a wurde ein Polyglycerin mit einem mittleren Kondensationsgrad n̄ = 2,1 (OH-Zahl = 1364 mgKOH/g) in den Polyglycerin-Maleinsäure-Ester überführt. Die erhaltene hellgelbe 70 %ige wässrige Lösung von PGN-MSA-Ester hatte eine Verseifungszahl von 800 mgKOH/g und eine Säurezahl von 444 mgKOH/g (jeweils berechnet 100 %). Der Gehalt an freier Maleinsäure betrug nach HPLC-Analyse 3,7 %. Die Esterlösung wurde analog zu Beipsiel 1b mit Iminodiessigsäure-Natrium umgesetzt.

Beispiel 4

Ein Polyglycerin-Maleinsäure-Ester wurde analog zu Beispiel 1a hergestellt. Das resultierende gelbe Öl wurde jedoch nicht wie dort mit Wasser versetzt, sondern wie folgt mit IDA umgesetzt. 226 g (1,7 mol) IDA wurden in 250 g Wasser suspendiert. Die Suspension wurde mit 277 g 40 %ige wäßrige NaOH auf einen pH-Wert von 10 gestellt. Nach Zugabe von 90 g Soda wurden in einem Temperaturbereich von 10 bis 15 °C 259 g des oben hergestellten OGN-MS-Esters innerhalb von 30 Minuten portionsweise unter kräftigem Rühren zugegeben. Hierbei wurde der pH-Wert durch Zutropfen von 235 g 40 %iger wäßriger NaOH bei 10 gehalten. Anschließend wurde 3 Stunden bei 25 °C weitergerührt.
HPLC-Daten der resultierenden Lösung:
3,5 % IDA-Natrium, 2,8 % Asparaginsäurediessigsäure-Natrium, 1,5 % Maleinsäure- und 2,1 % Fumarsäure-Natrium.

Nach Zugabe von 1,5 l Methanol wurde der resultierende Niederschlag abgesaugt und getrocknet.
HPLC: 3,9 % IDA, 3,8 % Asparaginsäurediessigsäure, 0,5 % Maleinsäure, 0,25 % Fumarsäure, jeweils als Natriumsalz. Hydrierzahl: 12.

Beispiel 5

66,5 g (0,5 mol) Asparginsäure wurden in 70 g Wasser suspendiert. Mit 77 g 40 %iger wäßriger Natronlauge wurde ein pH-Wert von 9,5 eingestellt. Nach Zugabe von 25 g Natriumcarbonat wurden innerhalb von 30 Minuten unter kräftigem Rühren 118 g einer 70 %igen wäßrigen PGN-MS-Mischung, die gemäß Beispiel 1a) synthetisiert wurde, bei 10 bis 15°C zugetropft. Währenddessen wurde der pH-Wert durch Zusatz von 72 g 40 %iger wäßriger NaOH bei 9,5 gehalten. Die Reaktionsmischung wurde anschließend 2 Stunden bei 25°C weitergerührt. Die resultierende Na-Salzlösung wurde bei 10 bis 15°C mit 50 %iger Schwefelsäure neutralisiert und anschließend sprühgetrocknet.

Das sprühgetrocknete Produkt bestand zu 60 % aus dem mit Iminodibernsteinsäure formal veresterten Polyglycerinderivat der Formel I mit
R =

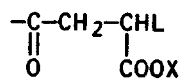

L =

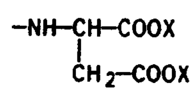

X = Na und
n = 2,8

Beispiel 6

69 g eines analog zu Beispiel 1a synthetisierten PGN-MS-Esters in 70 %iger wäßriger Lösung wurden bei 15 bis 20°C unter kräftigem Rühren mit 42,3 g Ethanolaminoessigsäure-Na-Salz (0,3 mol) versetzt. Nach Zugabe von 40 g Wasser wurden bei 10 bis 15°C portionsweise innerhalb von 10 Minuten 16 g Natriumcarbonat zugegeben und anschließend 66 g 40 %ige wäßrige Natronlauge innerhalb von 30 Minuten zugetropft. Es resultierte ein pH-Wert von 10. Das Reaktionsgemisch wurde anschließend 2,5 Stunden bei 25°C gerührt. Es resultierte eine klare wäßrige Natriumsalzlösung.

Das Produkt der Formel I mit
R =

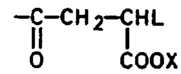

L =

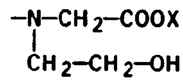

X = Na und
n = 2,8
wurde durch Zugabe von 700 ml Methanol ausgefällt, filtriert, mit Methanol gewaschen und getrocknet. Es enthielt jeweils als Na-Salze 1,3 % Ethanolaminoessigsäure, 2,8 % des formalen Adduktes von Ethanolaminoessigsäure an Maleinsäure, 0,3 % Fumarsäure und 1,5 % Maleinsäure. Die Ausbeute betrug 213 g.

Beispiel 7

Beispiel 6 wurde analog mit Sarkosin-Natrium anstelle von Ethanolaminoessigsäure-Natrium wiederholt. Nach Methanolfällung und Trocknung erhielt man ein Produkt der Formel I mit
R =

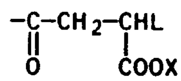

L =

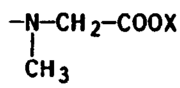

X = Na und
n = 2,8
Es enthält außerdem noch 2,3 % Sarkosin, 3,8 % des formalen Adduktes von Sarkosin an Maleinsäure, 0,3 % Fumarsäure und 1,6 % Maleinsäure jeweils in der Natriumsalzform.

Beispiel 8

169 g (1,0 Mol) Glutaminsäure-Natriumsalz wurden in 220 g Wasser gelöst. Nach Zugabe von 53 g Soda wurden bei einer Temperatur von 10 bis 15°C unter kräftigem Rühren 217 g einer 70 %igen Lösung eines analog zu Beispiel 1a synthetisierten PGN-MS-Esters innerhalb von 30 Minuten zugetropft.

Gleichzeitig wurde durch Zugabe von insgesamt 187 g 25 %iger Natronlauge der pH-Wert bei 9,5 gehalten. Anschließend rührte man das Reaktionsgemisch 2,5 Stunden bei 25°C. Es resultierte eine klare wäßrige Natriumsalzlösung von PGN-MS-Glutamat der Formel I, in der

R = 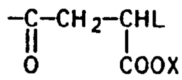 , L = 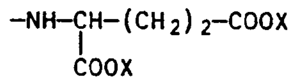 ,

X = Na und n ≙ 2,8 bedeutet. Das Additionsprodukt von Glutaminsäure-Natriumsalz an den obengenannten PGN-MS-Ester wurde durch Zugabe von 900 ml Methanol ausgefällt, filtriert, mit Methanol gewaschen und getrocknet. Es enthielt als Verunreinigung, jeweils in Form der Na-Salze, 5,3 % Glutaminsäure, 4,1 % des formalen Adduktes von Glutaminsäure an Maleinsäure, 0,8 % Fumarsäure und 2,8 % Maleinsäure. Bei dem so hergestellten Additionsprodukt (PGN-MS-Glutamat) handelt es sich um einen wirksamen Komplexbildner für Schwermetall- und Erdalkalimetallionen, der sehr gut biologisch abbaubar ist.

Anwendungstechnischer Teil

Bestimmung der Calciumcarbonat-Dispergierkapazität

Die Calciumcarbonat-Dispergierkapazität wird dadurch bestimmt, daß man 1 g des Polymerisates in 100 ml destilliertem Wasser löst, bei Bedarf durch Zugabe von 1 g Natronlauge neutral stellt und mit 10 ml 10 %iger Sodalösung versetzt. Die Lösung wird anschließend mit 0,25 m Calciumacetatlösung titriert, wobei pH-Wert und die Temperatur konstant gehalten werden. Der pH-Wert wird entweder durch Zugabe von verdünnter Natronlauge- oder Salzsäurelösung eingestellt. Die Dispergierkapazität wird bei 20 °C und pH 11 sowie bei 80 °C und pH 10 bestimmt. Die Ergebnisse sind in folgender Tabelle angegeben:

| Polymerisat | Produkt von Beispiel-Nr. | Dispergierkapazität des Polymerisates [mg $CaCO_3$/g Polymerisat-Na-salz] | |
|---|---|---|---|
| | | 20 °C/pH 11 | 80 °C/pH 10 |
| Oligoglycerin/ MSA/IDA | 3 | 180 | 130 |
| Oligoglycerin/ MSA/IDA | 1 | 210 | 120 |
| Oligoglycerin/ MSA/IDA | 2 | 190 | 90 |
| Oligoglycerin/ MSA/IDA | 4 | 180 | 80 |
| Homopolyacrylat K-Wert 30 | Vergleich | 115 | 55 |

Bestimmung des Clay-Dispergiervermögens

In einen 100 ml Meßzylinder (graduiert) werden 1 g China-Clay (SPS 151), 100 ml destilliertes Wasser und 10 ppm Polymer (als 100 % Natrium-salz) gegeben. Die Dispersion wird mit einem geeigneten Rührgerät intensiv durchmischt und anschließend 3 Stunden gelagert. Nach 3 Stunden nimmt man aus der Mitte des Meßzylinders eine Probe von 2,5 ml, verdünnt mit destilliertem Wasser auf 25 ml und bestimmt die Trübung der Probe mittels eines Photometers. Die Trübung wird in NTU-Einheiten (normal turbidity units) angegeben. Je stabiler die Dispersion ist, um so höher liegen die erhaltenen NTU-Werte.

| Polymerisat | Produkt von Beispiel-Nr. | Trübungseinheiten [NTU] |
|---|---|---|
| ohne | | 50 |
| Oligoglycerin/ MSA/IDA | 4 | 360 |
| Oligoglycerin/ MSA/IDA | 1 | 370 |
| Oligoglycerin/ MSA/IDA | 3 | 350 |
| Oligoglycerin/ MSA/IDA | 2 | 350 |
| Homopolyacrylat K-Wert 30 | | 300 |

Sowohl bei der Calciumcarbonat-Dispergierkapazität als auch bei der Clay-Dispergierung werden deutliche Verbesserungen gegenüber dem den Stand der Technik (Homopolyacrylat) erhalten.

## Patentansprüche

1. Glycerinaminocarboxylate der Formel

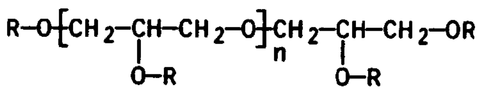

(I),

in der
n = 0 bis 10 bedeutet und
R für

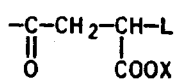

oder

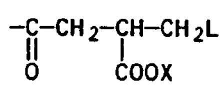

steht, wobei
L einen Iminodiacetat-, Aspartat-, Glutamat-, Sarcosinat-, Glycinat-, Serinat-, Hydroxyaspartat-, Ethanolaminoacetat-, Diethanolamino-, Alanat- oder Taurinatrest darstellt und

X für Wasserstoff-, Alkalimetall-, Ammonium- oder substituiertes Ammoniumion steht.

2. Glycerinaminocarboxylate der Formel I nach Anspruch 1, in der L einen Iminodiacetatrest darstellt und n = 1 bis 4 ist.

3. Verfahren zur Herstellung von Verbindungen der Formel I nach Anspruch 1, dadurch gekennzeichnet, daß man

(a) Verbindungen der Formel

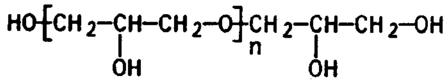 (II),

in der n = 0 bis 10 bedeutet, mit

(b) Maleinsäureanhydrid, Itaconsäureanhydrid, einem Maleinsäurehalbester oder einem Itaconsäurehalbester zu Verbindungen der Formel

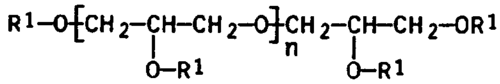 (III),

in der

$R^1$ für

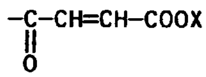 oder 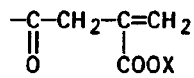

steht und X die in Formel I angegebene Bedeutung hat, verestert und anschließend die Verbindungen der Formel III mit Verbindungen der Formel

L - H (IV),

in der L die im Anspruch 1 angegebene Bedeutung hat, im schwach alkalisch wässrigen Milieu zu Verbindungen der Formel I umsetzt.

4. Verfahren nach Anspruch 3, dadurch gekennzeichnet, daß man die Verbindungen der Formel II in Abwesenheit von Lösemitteln mit Maleinsäureanhydrid in Form einer viskosen Schmelze verestert, dann soviel Wasser zufügt, daß eine 50 bis 80 gew.%igen wäßrigen Lösung entsteht und diese Lösung dann mit Verbindungen der Formel IV zu Verbindungen der Formel I umsetzt.

5. Verfahren nach Anspruch 3, dadurch gekennzeichnet, daß man Verbindungen der Formel II bei Temperaturen von 80 bis 140°C unter Ausschluß von Wasser verestert, dann soviel Wasser zufügt, daß eine 50 bis 80 gew.-%ige wäßrige Lösung entsteht und durch Zugabe von Glutaminsäure zu Verbindungen der Formel I umsetzt.

6. Verfahren nach Anspruch 3 oder 4, dadurch gekennzeichnet, daß man die Umsetzung von III mit IV zu I bei pH-Werten von 7,5 bis 11 und bei Temperaturen von 10 bis 70°C durchführt.

7. Verfahren nach Anspruch 3 oder 4, dadurch gekennzeichnet, daß man die Umsetzung von Verbindungen der Formel III mit den Verbindungen der Formel IV bei pH-Werten von 9 bis 10 und Temperaturen von 10 bis 40°C durchführt.

8. Verwendung von Verbindungen der Formel I gemäß Anspruch 1 als Komplexbildner für Schwermetall- und/oder Erdalkalimetallionen.

9. Verwendung nach Anspruch 8, dadurch gekennzeichnet, daß man die Verbindungen der Formel I als Komplexbildner in wäßrigen Bädern für die chemische Abscheidung von Kupfer einsetzt.

## Claims

1. A glycerol aminocarboxylate of formula

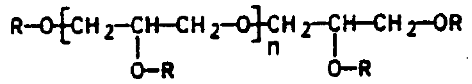

(I)

where

n is from 0 to 10 and

R is

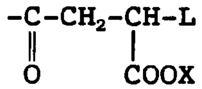 or 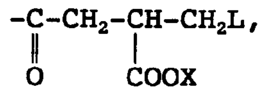

where

L is iminodiacetate, aspartate, glutamate, sarcosinate, glycinate, serinate, hydroxyaspartate, ethanolaminoacetate, diethanolamino, alanate or taurinate and

X is hydrogen, an alkali metal, ammonium or substituted ammonium.

**2.** A glycerol aminocarboxylate of formula I as claimed in claim 1, where L is iminodiacetate and n is from 1 to 4.

**3.** A process for preparing a compound of the formula I as claimed in claim 1, which comprises esterifying (a) a compound of formula

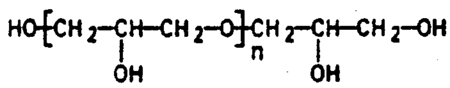

(II)

where n is from 0 to 10, with

(b) maleic anhydride, itaconic anhydride, a maleic half-ester or an itaconic half-ester to give a compound of the formula

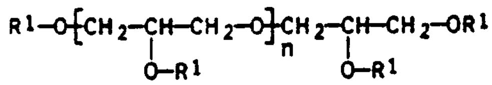

(III)

where

$R^1$ is

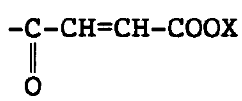 or 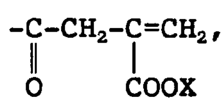

and X is as defined in the formula I, and then reacting the compound of the formula III with a compound of the formula

L - H (IV)

where L is as defined in claim 1, in a weakly alkaline aqueous medium to give a compound of the formula I.

**4.** A process as claimed in claim 3, wherein the compounds of the formula II are esterified with maleic anhydride in the absence of solvents in the form of a viscous melt, then sufficient water is added to form an aqueous solution from 50 to 80% strength by weight, and this solution is then reacted with compounds of the formula IV to form compounds of the formula I.

5. A process as claimed in claim 3, wherein compounds of the formula II are esterified at from 80 to 140°C in the absence of water, then sufficient water is added to form an aqueous solution from 50 to 80% strength by weight, and glutamic acid is added to form compounds of the formula I.

6. A process as claimed in claim 3 or 4, wherein the reaction of III with IV to give I is carried out at pH 7.5-11 and at 10-70°C.

7. A process as claimed in claim 3 or 4, wherein the reaction of a compound of the formula III with a compound of the formula IV is carried out at pH 9-10 and 10-40°C.

8. The use of compounds of the formula I as claimed in claim 1 as complexing agents for heavy metal and/or alkaline earth metal ions.

9. A use as claimed in claim 8, wherein the compounds of the formula I are used as complexing agents in aqueous baths for the chemical deposition of copper.

## Revendications

1. Aminocarboxylates de glycérine de la formule

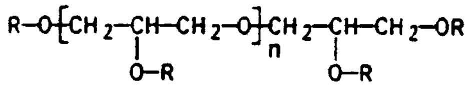

(I),

dans laquelle
n a une valeur qui fluctue de 0 à 10 et
R représente un groupe,

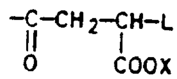

ou

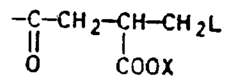

,

où
L représente un radical iminodiacétate, aspartate, glutamate, sarcosinate, glycinate, sérinate, hydroxyaspartate, éthanolaminoacétate, diéthanolamino-, alanate ou taurinate et
X représente un atome d'hydrogène, un ion métal alcalin, ammonium ou ammonium substitué.

2. Aminocarboxylates de glycérine de la formule I suivant la revendication 1, dans lesquels L représente un radical iminodiacétate et n a une valeur qui varie de 1 à 4.

3. Procédé de préparation de composés de la formule I suivant la revendication 1, caractérisé en ce que
(a) on estérifie des composés de la formule

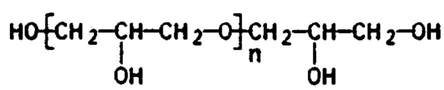

(II),

dans laquelle n a une valeur qui varie de 0 à 10, avec
(b) l'anhydride maléique, l'anhydride itaconique, un hémiester de l'acide maléique ou un hémiester de l'acide itaconique, de manière à obtenir des composés de la formule

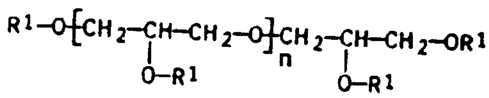

(III),

dans laquelle

$R^1$ représente un radical

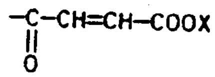 ou 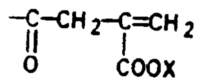

et X possède les signfications qui lui ont été attribuées à propos de la définition de la formule I et on fait ensuite réagir les composés de la formule III avec des composés de la formule

L - H (IV),

dans laquelle L possède les significations qui lui ont été attribuées dans la revendication 1 en milieu aqueux faiblement alcalin, de manière à obtenir les composés de la formule I.

4. Procédé suivant la revendication 3, caractérisé en ce que l'on estérifie les composés de la formule II en l'absence de solvant avec l'anhydride maléique sous forme d'une masse fondue visqueuse, puis on ajoute de l'eau en suffisance pour qu'une solution aqueuse à 50 à 80% en poids prenne naissance et on fait ensuite réagir cette solution avec des composés de la formule IV de manière à obtenir des composés de la formule I.

5. Procédé suivant la revendication 3, caractérisé en ce que l'on estérifie des composés de la formule II à des températures de 80 à 140°C, en l'absence d'eau, puis on ajoute de l'eau en suffisance pour qu'une solution aqueuse à 50-80% en poids prenne naissance et on les convertit en composés de la formule I par addition d'acide glutamique.

6. Procédé suivant la revendication 3 ou 4, caractérisé en ce que l'on entreprend la réaction des composés de la formule III avec les composés de la formule IV à des températures de 10 à 70°C et à des valeurs du pH de 7,5 à 11.

7. Procédé suivant la revendication 3 ou 4, caractérisé en ce que l'on entreprend la réaction des composés de la formule III avec les composés de la formule IV à des températures de 10 à 40°C et à des valeurs du pH de 9 à 10.

8. Utilisation de composés de la formule I suivant la revendication 1 à titre d'agents de complexation pour des ions de métaux lourds et/ou de métaux alcalino-terreux.

9. Procédé suivant la revendication 8, caractérisé en ce que l'on exploite les composés de la formule I à titre d'agents de complexation dans des bains aqueux en vue de la séparation chimique de cuivre.